(19) Europäisches Patentamt
European Patent Office
Office européen des brevets

(11) Veröffentlichungsnummer : **0 167 021**
**B1**

(12) # EUROPÄISCHE PATENTSCHRIFT

(45) Veröffentlichungstag der Patentschrift : **30.12.86**

(21) Anmeldenummer : **85107112.6**

(22) Anmeldetag : **10.06.85**

(51) Int. Cl.⁴ : **C 07 C 45/67**, C 07 C 47/21, C 07 C 47/22, C 07 C 47/232

(54) **Verfahren zur Herstellung von 2-Methyl-2-alkenalen.**

(30) Priorität : **12.06.84 DE 3421809**

(43) Veröffentlichungstag der Anmeldung : **08.01.86 Patentblatt 86/02**

(45) Bekanntmachung des Hinweises auf die Patenterteilung : **30.12.86 Patentblatt 86/52**

(84) Benannte Vertragsstaaten : **BE CH DE FR GB IT LI NL**

(56) Entgegenhaltungen :
**EP-A- 0 012 570**
**FR-A- 2 386 509**

(73) Patentinhaber : **BASF Aktiengesellschaft**
**Carl-Bosch-Strasse 38**
**D-6700 Ludwigshafen (DE)**

(72) Erfinder : **Fischer, Rolf, Dr.**
**Bergstrasse 98**
**D-6900 Heidelberg (DE)**
Erfinder : **Hoelderich, Wolfgang, Dr.**
**Mannheimer Strasse 18c**
**D-6710 Frankenthal (DE)**
Erfinder : **Merger, Franz, Dr.**
**Max-Slevogt-Strasse 25**
**D-6710 Frankenthal (DE)**
Erfinder : **Mross, Wolf Dieter, Dr.**
**Anselm-Feuerbach-Strasse 21**
**D-6710 Frankenthal (DE)**
Erfinder : **Weitz, Hans-Martin, Dr.**
**Auf dem Koeppel 40**
**D-6702 Bad Duerkheim (DE)**

**EP 0 167 021 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 2-Methyl-2-alkenalen durch Isomerisierung von Acroleinderivaten an zeolithischen Katalysatoren.

Aus der DE-OS 26 21 224 ist bekannt, daß man 4-Acetoxy-2-formyl-2-buten (4-Acetoxytiglinaldehyd) erhält, wenn man bei erhöhter Temperatur Wasserstoff durch eine Lösung von 4-Acetoxy-2-formyl-1-buten leitet, in der ein durch Schwefel dotierter Palladium-Trägerkatalysator suspendiert ist. Man isoliert Gemische aus 4-Acetoxy-2-formyl-2-buten und 4-Acetoxy-2-formyl-butan im Verhältnis vier zu eins. Diese Isomerisierung an Palladium-Katalysatoren hat den Nachteil, daß beträchtliche Mengen des Ausgangsprodukts zu dem nicht verwendbaren 4-Acetoxy-2-formylbutan hydriert werden.

In Liebigs Ann. 494, 273 (1932) wird beschrieben, daß man durch Behandlung von α-Ethylacrolein mit Calciumchlorid und anschließende Destillation Tiglinaldehyd (trans-2-Methyl-2-butenal) erhält. Diese Umlagerung konnte bei der Nacharbeitung der Literaturangaben nicht bestätigt werden.

Eine Umwandlung von 2-Alkylacroleinen in die entsprechenden 2-Methyl-2-alkenale kann man auch erreichen, wenn man zunächst die jeweiligen 2-Alkylacroleindialkylhydrazone herstellt, diese dann mit katalytischen Mengen starker Säuren in die Dialkylhydrazone der entsprechenden 2-Methyl-2-alkenale umlagert und zuletzt aus den Hydrazonen die 2-Methyl-2-alkenale durch Hydrolyse freisetzt (Zh. Org. Khim. 5, 1183 (1969). Man benötigt somit drei Reaktionsschritte. Außerdem fällt das eingesetzte Dialkylhydrazin im letzten Reaktionsschritt als Salz an und muß daher mit Alkali unter Neutralsalzbildung freigesetzt werden.

Es wurde nun gefunden, daß sich 2-Methyl-2-alkenale der Formel

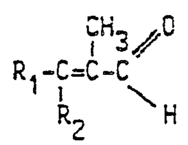

(I)

in der $R^1$ und $R^2$ Wasserstoffatome, Alkylreste mit 1 bis 18 Kohlenstoffatomen oder aromatische Reste bedeuten, erheblich vorteilhafter dadurch herstellen lassen, daß man Acroleinderivate der Formel

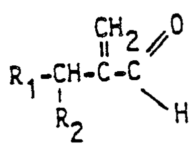

(II)

in der $R^1$ und $R^2$ die obengenannte Bedeutung haben, bei Temperaturen von 30 bis 450 °C mit zeolithischen Katalysatoren, Aluminiumsilikaten und/oder Aluminiumphosphaten in Berührung bringt.

Das erfindungsgemäße Verfahren läßt sich z. B. für die Isomerisierung von Ethylacrolein zu Tiglinaldehyd (trans-2-Methyl-2-butenal) durch die folgenden Formeln wiedergeben :

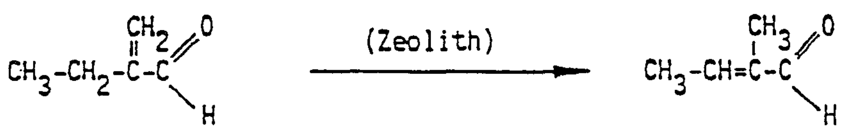


Die als Ausgangsstoffe verwendeten Acroleinderivate der Formel II enthalten als Reste $R^1$ und $R^2$ Wasserstoffatome, Alkylreste mit 1 bis 18 Kohlenstoffatomen und aromatische Reste. Alkylreste sind z. B. Methyl-, Ethyl-, n-Propyl-, i-Propyl-, n-Butyl-, i-Butyl-, tert.-Butyl-, Pentyl-, Decyl- oder Dodecylreste. Als aromatische Reste kommen z. B. Phenylreste in Frage, die gegebenenfalls noch durch andere Reste, wie Alkyl- oder Alkoxyreste oder durch Halogenatome substituiert sein können.

Beispielsweise seien die folgenden Verbindungen der Formel II genannt : 2 Ethylacrolein, 2-n-Butylacrolein, 2-i-Propylacrolein, 2-n-Propylacrolein, 2-Decylacrolein, 2-n-Pentylacrolein, 2-Benzylacrolein, 2-Heptylacrolein, 2-n-Hexylacrolein, 2-i-Butylacrolein, 2-n-Nonylacrolein. Die Ausgangsverbindungen der Formel II lassen sich z. B. durch Umsetzung von Alkanalen mit Formaldehyd und sekundären Aminen in Gegenwart von Carbonsäuren herstellen (DT-OS 31 06 557).

Als Katalysatoren werden für die erfindungsgemäße Isomisierung der Acroleinderivate II z. B. Zeolithe verwendet. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2. Die Electrovalenz der Aluminium enthaltenden Tetraeder ist durch Einschluß von Kationen in den Kristall z. B. eines Alkali- oder Wasserstoffions ausgeglichen. Ein Kationenaustausch ist möglich. Die Räume

zwischen den Tetraedern sind vor der Dehydratation durch Trocknen bzw. Calcinieren von Wassermolekylen besetzt. Auch gibt es kristalline Verbindungen mit Zeolithstruktur, bei denen dreiwertige Elemente wie B, Ga, Fe, Cr anstelle des Aluminiums oder vierwertige Elemente wie Ge anstelle des Siliciums in das Zeolithgitter eingebaut sind.

Vorzugsweise werden Zeolithe des Pentasiltyps eingesetzt. Diese Zeolithe können unterschiedliche chemische Zusammensetzung aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische.

Insbesondere sind die Alumino-, Boro- und Eisensilikatzeolithe des Pentasiltyps geeignet. Der Aluminosilikatzeolith wird aus einer Aluminiumverbindung, vorzugsweise $Al(OH)_3$ oder $Al_2(SO_4)_3$, und einer Siliciumkomponente, vorzugsweise hochdispersem Siliciumdioxid in wäßriger Aminlösung, insbesondere in 1,6-Hexandiamin- oder 1,3-Propandiamin- oder Triethylen-tetramin-Lösung mit und ohne Alkali- oder Erdalkalizusatz bei 100 bis 220 °C unter autogenem Druck hergestellt. Die erhaltenen Aluminosilikatzeolithe enthalten je nach Wahl der Einsatzstoffmengen ein $SiO_2/Al_2O_3$-Verhältnis von 10 bis 40 000. Derartige Aluminosilikatzeolithe lassen sich auch in etherischem Medium, wie in Diethylenglykoldimethylether, in alkoholischem Medium, wie in Methanol bzw. 1,4-Butandiol oder lediglich in Wasser synthetisieren.

Der Borosilikatzeolith wird bei 90 bis 200 °C unter autogenem Druck synthetisiert, indem man eine Borverbindung, z. B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und ohne Alkali- oder Erdalkalizusatz zur Reaktion bringt. Solche Borosilikatzeolithe können auch dadurch hergestellt werden, daß man die Reaktion statt in wäßriger Aminlösung in etherischer Lösung, z. B. Diethylenglykoldimethylether, oder in alkoholischer Lösung, z. B. in 1,6-Hexandiol, durchführt. Den Eisensilikatzeolith erhält man aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit und ohne Alkali- oder Erdalkalizusatz bei 100-220 °C unter autogenem Druck.

Die so hergestellten Alumino-, Boro- und Eisensilikatzeolithe können nach ihrer Isolierung, Trocknung bei 100 bis 160 °C, vorzugsweise 110 °C, und Calcination bei 450 bis 550 °C, vorzugsweise 500 °C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 96 : 5, bevorzugt 75 : 25, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge bei 110 °C/16 h getrocknet und bei 500 °C/16 h calciniert. Man erhält auch vorteilhafte Katalysatoren, wenn der isolierte Alumino-bzw. Borosilikatzeolith direkt nach der Trocknung verformt wird und erstmals nach der Verformung einer Calcination unterworfen wird. Die Alumino-, Boro- und Eisensilikatzeolithe können aber auch in reiner Form, ohne Binder, als Stränge oder Tabletten eingesetzt werden.

Weiterhin können Aluminosilikatzeolithe des Y-Typs verwendet werden, die aus Kieselsol (29 % $SiO_2$) und Natriumaluminat in wäßrigem Medium hergestellt werden. Diese Aluminosilikatzeolithe können vor ihrem Einsatz ebenfalls mit Bindern verformt werden. Auch Zeolithe des Mordenit-Typs und des X-Typs sind geeignet.

Liegt der Zeolith von der Synthese her nicht in der katalytisch aktiven, aciden H-Form vor, sondern z. B. in der Na-Form, dann kann diese durch Ionenaustausch mit Ammoniumionen und anschließende Calcination oder durch Behandlung mit Säuren vollkommen oder partiell in die gewünschte H-Form übergeführt werden.

Da bei der erfindungsgemäßen Verwendung der zeolithischen Katalysatoren eine durch Koksabscheidung bedingte Desaktivierung eintreten kann, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Zur Erhöhung der Selektivität, der Standzeit und der Anzahl an Regenerierungen kann es nützlich sein, Modifizierungen an den zeolithischen Katalysatoren vorzunehmen. Eine geeignete Modifizierung besteht darin, daß man den unverformten oder verformten Zeolithen mit Alkalimetallen, wie Natrium, sofern nicht schon von der Synthese her die Alkali-Form des Zeolithen vorliegt, mit Erdalkalimetallen, wie Ca, Mg, oder Erdmetallen, wie B, Tl, dotiert, z. B. durch einen Ionenaustausch oder durch Imprägnierung mit den entsprechenden Metallsalzen.

Durch eine Dotierung der Zeolithe mit Übergangsmetallen, wie W, Fe, Zn, mit Edelmetallen, wie Pd, und mit seltenen Erdmetallen, wie Ce, La, kann man besonders vorteilhafte Katalysatoren erhalten. Dabei kann man z. B. so verfahren, daß man den verformten Pentasilzeolithen in einem Steigrohr vorlegt und bei 20 bis 100 °C z. B. eine wäßrige Lösung eines Halogenids oder eines Nitrats der voranbeschriebenen Metalle überleitet. Ein derartiger Ionenaustausch kann z. B. an der Wasserstoff-, Ammonium- und Alkaliform des Zeolithen vorgenommen werden. Eine weitere Möglichkeit der Metallaufbringung auf den Zeolithen besteht darin, daß man das zeolithische Material z. B. mit einem Halogenid, einem Nitrat oder Oxid der vorbeschriebenen Metalle in wäßriger oder alkoholischer Lösung imprägniert. Sowohl an einen Ionenaustausch als auch an eine Imprägnierung schließt sich zumindest eine Trocknung, wahlweise eine

abermalige Calcination an.

Eine mögliche Modifizierung besteht darin, daß man den verstrangten oder unverstrangten Zeolith eine gewisse Zeit, ca. 30 min., mit einer Lösung von Wolframsäure $H_2WO_4$ oder $Ce(NO_3)_3 \cdot 6H_2O$ tränkt. Danach wird die überstehende Lösung am Rotationsverdampfer von Wasser befreit und der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z. B. eine ammoniakalische $Pd(NO_3)_2$-Lösung herzustellen und darin den reinen pulverförmigen Zeolithen bei 40-100 °C unter Rühren ca. 24 h aufzuschlämmen. Nach Abfiltrieren, Trocknen bei ca. 110 °C und Calcination bei ca. 500 °C kann das so gewonnene zeolithische Material mit und ohne Bindemittel zu Strängen oder Pellets weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z. B. eine ammoniakalische $Pd(NO_3)_2$-Lösung bei leicht erhöhter Temperatur zwischen 30 und 80 °C im Kreislauf 15-20 h leitet. Danach wird mit Wasser ausgewaschen, bei ca. 150 °C getrocknet und bei ca. 550 °C calciniert.

Bei manchen metalldotierten Zeolithen ist eine Nachbehandlung mit Wasserstoff vorteilhaft. Eine weitere Möglichkeit der Modifizierung besteht darin, daß man das zeolithische Material — verformt oder unverformt — einer Behandlung mit Säuren wie Salzsäure, Flußsäure und Phosphorsäure und/oder mit Wasserdampf unterwirft. Auch kann durch partielle Verkokung (pre-coke) die Aktivität des Katalysators für ein Selektivitätsoptimum des gewünschten Reaktionsproduktes eingestellt werden.

Als Katalysatoren für die erfindungsgemäße Isomerisierung der Acrylderivate der Formel II können auch Aluminosilikate und/oder Aluminiumphosphate eingesetzt werden.

Aluminosilikate sind Verbindungen aus $Al_2O_3$ und $SiO_2$. Derartige Verbindungen lassen sich beispielsweise aus $Al_2(SO_4)_3 \cdot 18H_2O$ und Wasserglas herstellen. Dabei verfährt man z. B. so, daß man das $Al_2(SO_4)_3 \cdot 18H_2O$ und Wasserglas bei pH 1 bis 3, bevorzugt pH 1,5, in Gegenwart von $H_2SO_4$ zusammengibt. Danach wird diese Mischung mit einer Ammoniak-Lösung bei pH 4 bis 8, bevorzugt 6, bei 0 bis 50 °C, bevorzugt bei 20 bis 30 °C, umgesetzt. Der hierbei entstandene Niederschlag wird abfiltriert, mit Ammoniumcarbonat-Lösung gewaschen und bei 50 bis 150 °C getrocknet. Besonders vorteilhafte Katalysatoren werden erhalten, wenn man ein $SiO_2 : Al_2O_3$-Verhältnis von 80 : 20 bis 20 : 80, insbesondere 75 : 25 bis 35 : 65, einstellt.

Aluminophosphate können beispielsweise durch Fällung aus $Al(NO_3)_3 \cdot 9H_2O$ mit $(NH_4)_3PO_4$ oder $(NH_4)_2HPO_4$ oder $(NH_4)H_2PO_4$ bei unterschiedlichen pH-Werten, insbesondere bei pH 3 bis pH 10, synthetisiert werden. Nach Abfiltrieren und Waschen wird das erhaltene Produkt bei 50 bis 150 °C, vorzugsweise 60 bis 100 °C, getrocknet und bei 300 bis 900 °C, vorzugsweise 500 bis 800 °C calciniert.

Die so hergestellten Aluminosilikate und Aluminophosphate können in reiner Form, ohne Bindemittel, zu Strängen, Tabletten oder Wirbelgut verarbeitet und eingesetzt werden. Man kann sie aber auch nach Isolierung, Trocknung und eventueller Calcination mit verschiedenen Bindern zu Strängen oder Tabletten verarbeiten. Das gewichtsmäßige Verhältnis Silikat bzw. Phosphat zu Bindemittel beträgt zweckmäßigerweise 90 : 10 bis 30 : 70. Als Bindemittel eignen sich diverse Aluminiumoxide, bevorzugt Boehmit, Siliciumdioxid, bevorzugt hochdisperses $SiO_2$, Gemische aus hochdispersem $SiO_2$ und hochdispersem $Al_2O_3$, hochdisperses $TiO_2$ sowie Ton. Nach der Verformung werden die Extrudate oder Preßlinge z. B. bei 110 °C/16 h getrocknet und bei 500 °C/16 h calciniert.

Auch diese Katalysatoren lassen sich nach einer Desaktivierung durch Koksabscheidung wie oben beschrieben regenerieren. Zur Erhöhung der Selektivität, der Standzeit und der Anzahl an Regenerierungen kann man auch diese Katalysatoren, wie oben für die Zeolithe angegeben, modifizieren und dotieren. Die Katalysatoren können wahlweise z. B. als 2 bis 4 mm Stränge oder als Tabletten mit 3 bis 5 mm Durchmesser oder als Pulver mit Teilchengröße von 0,1 bis 0,5 mm eingesetzt werden.

Man bringt die Acroleinderivate der Formel II mit den genannten Katalysatoren bei Temperaturen von 30 bis 500 °C in Berührung. Zweckmäßigerweise führt man die Umsetzung in einem Rieselreaktor durch. Man kann als Reaktionsgefäß aber auch einen Rührkolben oder Rührautoklaven verwenden. Man isomerisiert z. B. in der Gasphase bei 150° bis 450 °C, vorzugsweise bei 300° bis 400 °C und bei einer Belastung (WHSV) von 0,1 bis 20 $h^{-1}$, bevorzugt 0,5 bis 5 $h^{-1}$ (g Acroleinverbindung II/g Katalysator und Stunde). Man kann aber auch in der Flüssigphase bei Temperaturen von 30 bis 300 °C isomerisieren.

Das Verfahren kann chargenweise oder kontinuierlich, drucklos oder unter Druck durchgeführt werden. Unumgesetzte Ausgangsstoffe der Formel II können gegebenenfalls nach der Umsetzung destillativ von den entstandenen 2-Methyl-2-alkenalen I abgetrennt und erneut für die erfindungsgemäße Umsetzung verwendet werden.

Man kann die Isomerisierung in Gegenwart von Gasen, wie Wasserstoff, Stickstoff und Wasserdampf durchführen, wodurch die Produktzusammensetzung und die Standzeit des Katalysators beeinflußt wird. Insbesondere durch Wasserdampfzusatz läßt sich die Desaktivierung des Katalysators zurückdrängen.

Die nach dem erfindungsgemäßen Verfahren erhältlichen 2-Methyl-2-alkenale I stellen wertvolle Zwischenprodukte für die Herstellung von Farbstoffen, Pharmazeutika und Pflanzenschutzmitteln dar.

Beispiele 1 bis 14

Ethylacrolein wurde zum Zwecke der Isomerisierung in der Gasphase unter isothermen Bedingungen

in einem Rohrreaktor mit einem Innendurchmesser von 0,6 cm und einer Länge von 90 cm bei einer Temperatur zwischen 350 und 400 °C über den Katalysator geleitet. Die Versuchsdauer betrug jeweils etwa 6 Stunden. Die Art des Katalysators, die gewählte Temperatur, die Belastung (WHSV) und die Selektivität sind der folgenden Tabelle zu entnehmen.

| Beispiel | 1 | 2 | 3 | 4 | 5 | 6 | 7 | 8 | 9 | 10 | 11 | 12 | 13 | 14 |
|---|---|---|---|---|---|---|---|---|---|---|---|---|---|---|
| Katalysator | A | B | C | D | E | F | G | H | I | J | K | L | M | N |
| Temperatur [°C] | 350 | 400 | 400 | 400 | 400 | 400 | 400 | 400 | 400 | 400 | 400 | 400 | 400 | 400 |
| WHSV [$h^{-1}$] | 1,3 | 2,5 | 1,9 | 2 | 2 | 2 | 0,8 | 0,7 | 1,9 | 1,9 | 1,7 | 1,8 | 1,8 | 1,8 |
| Umsatz [%] | 20,8 | 14,8 | 17,5 | 17,4 | 12,0 | 26,3 | 9,7 | 29,1 | 11,9 | 16,1 | 21,2 | 36 | 13,9 | 12,2 |
| Selektivität [%] | | | | | | | | | | | | | | |
| Tiglinaldehyd | 47,1 | 77,7 | 73,7 | 79,3 | 67,5 | 76,4 | 89,6 | 76,6 | 84,0 | 82,1 | 72,2 | 61,1 | 48,9 | 43,4 |

Die erhaltenen Reaktionsprodukte wurden destillativ aufgearbeitet und durch Siedepunkt, Brechungsindex und NMR-Spektrum charakterisiert. Die quantitative Bestimmung von Ethylacrolein und Tiglinaldehyd erfolgte gaschromatographisch. Die verwendeten Katalysatoren wurden folgendermaßen hergestellt:

Katalysator A

Ein Aluminosilikatzeolith des Pentasil-Typs wurde unter hydrothermalen Bedingungen bei autogenem Druck und 150 °C aus 65 g hochdispersem $SiO_2$, 20,3 g $Al_2(SO_4)_3 \times 18H_2O$ in 1 kg einer wäßrigen 1,6-Hexandiamin-Lösung (Mischung 50 :50 Gew.%) in einem Rührautoklaven synthetisiert. Nach Abfiltrieren und Auswaschen wurde das kristalline Reaktionsprodukt bei 110 °C/24 g getrocknet und bei 500 °C/24 h calciniert. Dieser Aluminosilikatzeolith enthielt 91,6 Gew.% $SiO_2$ und 4,6 Gew.% $Al_2O_3$. Er wurde mit Boehmit im Gewichtsverhältnis 60 : 40 zu 2 mm-Strängen verformt, bei 110 °C/16 h getrocknet und bei 500 °C/24 h calciniert.

Katalysator B

Ein Borzeolith des Pentasil-Typs wurde in einer hydrothermalen Synthese aus 64 g hochdispersem $SiO_2$, 12,2 g $H_3BO_3$, 800 g einer wäßrigen 1,6-Hexandiamin-Lösung (Mischung 50 : 50 Gew.%) bei 170 °C unter autogenem Druck in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Borsilikatzeolith setzte sich zusammen aus 94,2 Gew.% $SiO_2$ und 2,32 Gew.% $B_2O_3$. Es wurden daraus durch Verformen mit Boehmit im Gewichtsverhältnis 60 : 40 2 mm-Stränge hergestellt, die bei 110 °C/16 h getrocknet und bei 500 °C/24 h calciniert wurden.

Katalysator C

Dieser Katalysator wurde aus einem Eisensilikatzeolith durch Verformung zu Strängen mit Boehmit im Gewichtsverhältnis 60 : 40 und anschließender Calcination bei 500 °C/16 h hergestellt. Der Eisensilikatzeolith des Pentasil-Typs wurde unter hydrothermalen Bedingungen bei autogenem Druck und 165 °C aus 273 g Wasserglas, gelöst in 253 g einer wäßrigen 1,6-Hexandiamin-Lösung (Mischung 50 : 50 Gew.%), und 31 g Eisensulfat, gelöst in 21 g 96-%iger Schwefelsäure und 425 g Wasser, in einem Rührautoklaven während 4 Tagen synthetisiert, danach abfiltriert, ausgewaschen, bei 100 °C/24 h getrocknet und bei 500 °C/24 h calciniert. Dieser Eisensilikatzeolith hat ein $SiO_2/Fe_2O_3$-Verhältnis von 17,7 und einen $Na_2O$-Gehalt von 0,62 Gew.%.

Katalysator D

Der Katalysator wurde aus einem NaY-Zeolithen, der mit Boehmit im Verhältnis 60 : 40 verstrangt war, durch Ionenaustausch mit $NH_4Cl$ bei 80 °C und Calcination bei 500 °C erhalten. Der Ammoniumaustausch mit anschließender Calcination wurde mehrmals wiederholt, bis der Rest-Natriumgehalt bei 0,21 Gew.% lag.

Katalysator E

Als Katalysator wurde der im Handel erhältliche Zeolith ®Zeolon 900 (Norton Chemical Process Products, U.S.A.) verwendet, der mit Siliciumdioxid im Verhältnis 60 : 40 zu 2 mm-Strängen verformt war.

Katalysator F

Katalysator B wurde mit einer wäßrigen gesättigten $H_2WO_4$-Lösung ca. 30 min. getränkt. Das Wasser der überstehenden Restlösung wurde am Rotationsverdampfer abgezogen. Danach wurde der Katalysator bei 130 °C getrocknet und bei 550 °C calciniert. Der Vorgang wurde wiederholt, bis der Katalysator einen W-Gehalt von 4 Gew.% aufwies.

Katalysator G

Katalysator G wurde wie Katalysator F, jedoch mit $Ce(NO_3)_3 \cdot 6H_2O$ hergestellt. Die Tränkung wurde auf einen Ce-Gehalt des Katalysators von 7,2 Gew.% eingestellt.

Katalysator H

Über den Katalysator B wurde eine ammoniakalische $Pd(NH_3)_4(NO_3)_2$-Lösung im Kreis gepumpt (65 ml/min). Danach wurde bei 110 °C getrocknet und bei 500 °C calciniert. Der Pd-Gehalt betrug 3,3 Gew.%.

Katalysator I

Katalysator B wurde bei 80 °C mit einer 20-%igen NaCl-Lösung ionenausgetauscht. Nach der Calcination bei 500 °C enthielt der Katalysator 0,28 Gew.% Na.

Katalysator J

Katalysator B wurde mit Magnesiumacetat ca. 30 min. getränkt, bei 110 °C getrocknet und 500 °C calciniert. Der Mg-Gehalt betrug 2,3 Gew.%.

Katalysator K

Katalysator C wurde mit einer 20-%igen $NH_4Cl$-Lösung bei 80 °C einem Ionenaustausch unterworfen und bei 500 °C calciniert. Der Vorgang wurde mehrmals wiederholt, bis der Katalysator nur noch 0,03 Gew.% Na aufwies.

Katalysator L

Dieser Katalysator ist ein Aluminosilikat mit einem $SiO_2 : Al_2O_3$-Verhältnis von 75 : 25. Zu seiner Herstellung wurden 589 g $Al_2(SO_4)_3 \cdot 18H_2O$ mit 989 g Wasserglas (27,3 Gew.% $SiO_2$-Gehalt) in Gegenwart von $H_2SO_4$ bei pH 1,5 zusammengegeben. Dann wurde mit 1 l Wasser verdünnt. Die so erhaltene Lösung und 677 g Ammoniak-Lösung wurden gleichzeitig, aber getrennt, während 25 min und unter Rühren bei 20 °C und pH 6 in eine Vorlage zu 2 l Wasser gegeben. Nach 1 Std. Rühren wurde der gebildete Niederschlag abfiltriert und mit 0,5 gew.%iger $(NH_4)_2CO_3$-Lösung gut ausgewaschen. Das Aluminosilikat mit einem Aschegehalt von 39,4 g/100 g wurde tablettiert und mit 110 °C/16 h getrocknet.

Katalysator M

Dieser Katalysator ist ein Aluminosilikat mit einem $SiO_2$-$Al_2O_3$-Verhältnis von 35 : 65. Er wurde wie Katalysator L hergestellt, wobei jedoch 153 g $Al_2(SO_4)_3 \cdot 18H_2O$ und 461 g Wasserglas verwendet wurden.

Katalysator N

Zu einer Lösung von 80,5 g $NH_4H_2PO_4$ in 300 ml Wasser wurde im Laufe einer Stunde eine Lösung von 262,5 g $Al(NO_3)_3 \cdot 9H_2O$ in 400 ml Wasser gegeben. Dabei wurde zur Aufrechterhaltung eines pH-Wertes von 4 Ammoniak-Lösung zugetropft. Der entstandene Niederschlag wurde 16 h nachgerührt, abfiltriert, mit 800 ml Wasser ausgewaschen und bei 60 °C 16 h getrocknet. Das bei 750 °C/3 h calcinierte Produkt enthielt 22,6 Gew.% Al und 23,9 Gew.% P. Das erhaltene Aluminophosphat wurde zu 5 mm Tabletten verpreßt, bei 110 °C/16 h getrocknet und bei 500 °C/16 h calciniert.

Beispiel 15

Zur Ermittlung des Einflusses der Temperatur auf den Umsatz und die Selektivität wurden die Beispiele 2, 12 und 14 bei den in der folgenden Tabelle angegebenen Temperaturen und Belastungen wiederholt.

| Katalysator | B | B | B | L | L | N | N |
|---|---|---|---|---|---|---|---|
| Temperatur [°C] | 300 | 350 | 400 | 350 | 400 | 350 | 400 |
| WHSV [$h^{-1}$] | 0,8 | 0,8 | 0,8 | 1,8 | 1,8 | 1,8 | 1,8 |
| Umsatz [%] | 20,6 | 27,8 | 47,8 | 28,2 | 36,0 | 7,0 | 12,2 |
| Selektivität [%] | | | | | | | |
| Tiglinaldehyd | 30,6 | 52,5 | 63,8 | 38,7 | 61,1 | 48,6 | 43,4 |

Beispiel 16

Zur Ermittlung des Einflusses der Belastungsänderung auf das Produktspektrum wurde Beispiel 15 mit Katalysator B bei einer Belastung WHSV von 2,5 ausgeführt. Im Vergleich zum Beispiel 15 wurden die aus der folgenden Tabelle ersichtlichen Ergebnisse erhalten.

| | | |
|---|---|---|
| WHSV | 0,8 | 2,5 |
| Umsatz % | 47,8 | 14,8 |
| Selektivität % | | |
| Tiglinaldehyd | 63,8 | 77,7 |

**Patentansprüche**

1. Verfahren zur Herstellung von 2-Methyl-2-alkenalen der Formel

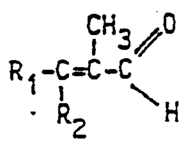

(I)

in der $R^1$ und $R^2$ Wasserstoffatome, Alkylreste mit 1 bis 18 Kohlenstoffatomen oder aromatische Reste bedeuten, dadurch gekennzeichnet, daß man Acroleinderivate der Formel

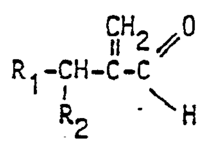

(II)

in der $R^1$ und $R^2$ die obengenannte Bedeutung haben, bei Temperaturen von 30 bis 500 °C mit zeolithischen Katalysatoren, Aluminiumsilikaten und/oder Aluminiumphosphaten in Berührung bringt.

2. Verfahren nach Anspruch 1, dadurch gekennzeichnet, daß man als Acroleinderivat Ethylacrolein verwendet.

3. Verfahren nach Anspruch 1 dadurch gekennzeichnet, daß man Zeolithe des Pentasil-Typs verwendet.

4. Verfahren nach Anspruch 1 und 3, dadurch gekennzeichnet, daß man Aluminosilikatzeolithe verwendet.

5. Verfahren nach Anspruch 1 und 3, dadurch gekennzeichnet, daß man Borosilikatzeolithe verwendet.

6. Verfahren nach Anspruch 1 und 3, dadurch gekennzeichnet, daß man Eisensilikatzeolithe verwendet.

7. Verfahren nach Anspruch 1, dadurch gekennzeichnet, daß man als Katalysatoren Aluminiumsilikatzeolithe des Y-Typs verwendet.

**Claims**

1. A process for the preparation of a 2-methylalk-2-enal of the formula

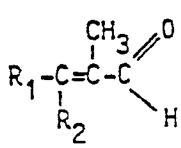

(I)

where $R^1$ and $R^2$ are each hydrogen, alkyl of 1 to 18 carbon atoms or an aromatic radical, wherein an acrolein derivative of the formula

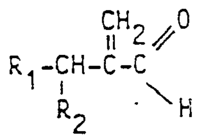

(II)

where $R^1$ and $R^2$ have the above meanings, is brought into contact with a zeolite catalyst, an aluminum silicate and/or an aluminum phosphate at from 30 to 500 °C.

2. A process as claimed in claim 1, wherein the acrolein derivative used is ethylacrolein.

3. A process as claimed in claim 1, wherein a zeolite of the pentasil type is used.

4. A process as claimed in claims 1 and 3, wherein an aluminosilicate zeolite is used.

5. A process as claimed in claims 1 and 3, wherein a borosilicate zeolite is used.

6. A process as claimed in claims 1 and 3, wherein an iron silicate zeolite is used.

7. A process as claimed in claim 1, wherein the catalyst used is an aluminosilicate zeolite of the Y type.

**Revendications**

1. Procédé de préparation de 2-méthyl-2-alcénals de formule

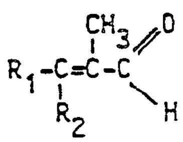

(I)

dans laquelle $R^1$ et $R^2$ sont des atomes d'hydrogène ou des radicaux alkyle ayant de 1 à 18 atomes de carbone ou aromatiques, caractérisé en ce qu'on met des dérivés d'acroléine de formule

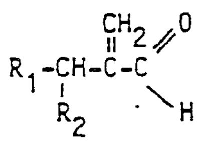

(II)

dans laquelle $R^1$ et $R^2$ sont tels que définis ci-dessus, en contact à une température de 30 à 500 °C avec un catalyseur zéolithique, un aluminosilicate et/ou un aluminophosphate.

2. Procédé selon la revendication 1, caractérisé en ce qu'on utilise l'éthylacroléine comme dérivé d'acroléine.

3. Procédé selon la revendication 1, caractérisé en ce qu'on utilise une zéolithe du type pentasil.

4. Procédé selon les revendications 1 et 3, caractérisé en ce qu'on utilise une zéolithe d'aluminosilicate.

5. Procédé selon les revendications 1 et 3, caractérisé en ce qu'on utilise une zéolithe de borosilicate.

6. Procédé selon les revendications 1 et 3, caractérisé en ce qu'on utilise une zéolithe de silicate de fer.

7. Procédé selon la revendication 1, caractérisé en ce qu'on utilise comme catalyseur une zéolithe d'aluminosilicate du type Y.